# EUROPÄISCHE PATENTSCHRIFT

(11) **EP 0 616 846 B1**
(45) Veröffentlichungstag und Bekanntmachung des Hinweises auf die Patenterteilung: **11.09.1996**
(21) Anmeldenummer: 94103487.8
(22) Anmeldetag: 08.03.1994
(51) Int. Cl.: B01J 21/18, B01J 37/02, B01J 37/12, C07B 35/02, B01J 23/40

(54) **Verfahren zur Herstellung edelmetallhaltiger Hydrierkatalysatoren auf Aktivkohle**
Process for the production of a noble metal hydrogenation catalyst supported on active carbon
Procédé de préparation d'un catalyseur d'hydrogénation à base de métal precieux déposé sur charbon actif

(30) Priorität: 15.03.1993 DE 4308101
(43) Veröffentlichungstag der Anmeldung: 28.09.1994
(73) Patentinhaber: Degussa Aktiengesellschaft, D-60311 Frankfurt (DE)
(72) Erfinder: Albers, Peter, Dr., D-63454 Hanau (DE); Burmeister, Roland, Dr., D-63826 Gieselbach (DE); Deller, Klaus, Dr., D-63512 Hainburg (DE); Despeyroux, Bertrand, Dr., F-78112 Fourqueux (FR)

(56) Entgegenhaltungen:
- EP-A- 0 289 725
- EP-A- 0 418 573
- EP-A- 0 573 910
- DE-A- 3 119 707
- US-A- 3 150 185

## Beschreibung

Die Erfindung betrifft ein Verfahren zur Herstellung edelmetallhaltiger Hydrierkatalysatoren, die wenigstens eine katalytisch aktive Komponente sowie gegebenenfalls Promotoren und/oder Modifikatoren auf einem Träger aus Aktivkohle enthalten.

Die Hydrierung von organischen Verbindungen wird großtechnisch in Gegenwart von edelmetallhaltigen Katalysatoren auf Aktivkohle durchgeführt. Ein wichtiges Anwendungsgebiet ist z. B. die Hydrierung von Halogenaminen. Aktivität und Selektivität dieser Katalysatoren können durch Promotoren und Modifikatoren beeinflußt werden. Promotoren verstarken die katalytische Wirkung, während Modifikatoren die Selektivität durch teilweise Vergiftung des Katalysators verändern. Je nach gewünschter Reaktion können bestimmte Komponenten in einem Fall als Promotoren und im anderen Fall als Modifikatoren wirken. Gemäß den Patentdokumenten DE-PS 21 50 220, USP 3,150,185, DE-OS 20 42 368 und der am Anmeldetag der vorliegenden Anmeldung noch nicht offengelegten deutschen Anmeldung P 42 18 866.0-41 (EP-A-573910) sind geeignete Edelmetalle die Metalle Pt, Pd, Rh, Ru, Os und Iridium. Als Modifikatoren werden S, Pb, Bi, Ag, Cu und Fe eingesetzt.

Als Träger für diese katalytisch wichtigen Komponenten wird bevorzugt Aktivkohle in feinteiliger Form mit einem mittleren Teilchendurchmesser von 15 bis 30 µm oder als Formkörper mit Abmessungen von 1 bis 5 mm eingesetzt.

Aktivkohle ist ein natürliches Produkt und wird durch Verkohlung mit nachfolgender chemischer oder Gas-Aktivierung aus kohlenstoffhaltigen Materialien gewonnen. Geeignete kohlenstoffhaltige Materialien sind verschiedene Hölzer wie Buchenholz, Pinienholz oder Kokosnußschalen (Ullmann's Encyclopedia of Industrial Chemistry, Weinheim 1985, Vol. A5, Seiten 124 - 140).

Aktivkohle zeichnet sich durch eine besonders hohe spezifische Oberfläche von 500 - 1500 m²/g aus, die aus Stickstoff-Adsorpttonstsothermen und Auswertung nach der Methode von Brunauer, Emmett und Teller (BET) bestimmt werden kann (DIN 66132). Die hohe spezifische Oberfläche von Aktivkohle beruht auf dem großen Porenvolumen von bis zu 1,5 ml/g, welches sich gewöhnlich zu mehr als einem Drittel aus Mikroporen mit Porendurchmessern unter 2 nm zusammensetzt.

Kommerzielle Aktivkohle enthält einen Aschenanteil von bis zu 20 %, der sich aus den mineralischen Komponenten der Ausgangsmaterialien zusammensetzt. Für katalytische Anwendungen können nur Aktivkohlen mit möglichst geringen Aschegehalten eingesetzt werden. Dies wird gewöhnlich durch Waschung der Aktivkohle mit starken Mineralsäuren wie zum Beispiel Salzsäure und Salpetersäure erreicht, siehe u. a. die europäishe Patentenmeldung EP-A-289725.

Dabei wird sowohl der Aschegehalt der Aktivkohleträger, insbesondere durch die Entfernung von erdalkali- und schwermetallhaltigen Verbindungen drastisch reduziert, als auch die Trägeroberfläche neu funktionalisiert. Diese beiden Eigenschaften wirken sich u. a. vorteilhaft auf den fertigen Katalysator hinsichtlich Katalysator-Aktivität und Produkt-Selektivität in den betroffenen chemischen Reaktionen aus. Typische Restaschegehalte liegen unter 2 Gew.-%.

Richard und Gallezot ("Preparation of highly dispersed, carbon supported, platinum catalyst" Stud. Surf. Sci. Catal., 31 (Prep. Catal. 4), 71 - 81; 1987, Elsevier Science Publishers B.V. Amsterdam) untersuchten die Funktionalisierung der Oberfläche von Kohlenstoffträgern mit verschiedenen Oxidationsmitteln. Sie fanden, daß die Oberfläche alternativ zu konzentrierter Salpetersäure (65 %) auch mit Natriumhypochlorit oder Wasserstoffperoxid funktionalisiert werden kann. Die Zahl der erhaltenen funktionellen Gruppen war jedoch bei Verwendung von Wasserstoffperoxid selbst bei Anwendung in 30 %iger wäßriger Lösung für die Dauer von 24 Stunden wesentlich geringer als bei Anwendung von konzentrierter Salpetersäure.

Zur Herstellung von Hydrierkatalysatoren wird von Aktivkohlen ausgegangen, deren Restaschegehalt durch eine Salpetersäurewäsche auf unter 2 Gew.-% vermindert wurde. Auf diesen Aktivkohlen müssen geeignete Edelmetalle abgeschieden werden. Dazu wird zum Beispiel, wie in der P 42 18 866.0-41 beschrieben, die pulverförmige Aktivkohle in Wasser suspendiert. Nach Zugabe wäßriger Lösungen von wasserlöslichen Verbindungen der Edelmetalle und gegebenenfalls der Modifikatoren zu der Aktivkohle-Suspension werden Edelmetalle und Modifikatoren auf der Aktivkohle in Form ihrer schwer löslichen Verbindungen durch Zugabe einer Base zur Suspension niedergeschlagen. Anschließend folgt gewöhnlich eine Reduktion der katalytisch wichtigen Komponenten mit einem Reduktionsmittel wie Hydrazin, Natriumformiat, Natriumboranat oder Formaldehyd.

Es hat sich gezeigt, daß die so hergestellten Hydrierkatalysatoren sehr große Schwankungen der Hydrieraktivität sowie der Selektivität aufweisen. Dies kann auf Schwankungen der Qualität dieser aus natürlichen Quellen stammenden Aktivkohleträger zurückgeführt werden.

Insbesondere sind der geringe Aschegehalt sowie die funktionalisierte Oberfläche nicht immer unter wirtschaftlich vertretbaren, großtechnisch durchgeführten Bedingungen der Salpetersäurewäsche einstellbar.

Allgemein zeichnet sich ein Optimum der Wäschebedingungen für einen bestimmten Aktivkohle-Typ ab. Erfahrungsgemäß kann nach Wäschen unter zu hohen Salpetersäurekonzentrationen und zu langen Waschzeiten die Leistung der resultierenden Katalysatoren negativ beeinflußt werden.

Aufgabe der vorliegenden Erfindung ist es, ein Verfahren zur Herstellung von Hydrierkatalysatoren auf Aktivkohleträgern anzugeben, welches die beobachteten Schwankungen der katalytischen Aktivität verringert und zudem aktivere Katalysatoren liefert.

Diese Aufgabe wird durch ein Verfahren zur Herstellung von edelmetallhaltigen Hydrierkatalysatoren gelöst, die auf einem Träger aus mit Salpetersäure gewaschener Aktivkohle wenigstens eine katalytisch aktive Komponente sowie gegebenenfalls Promotoren und/oder Modifikatoren enthalten.

Das Verfahren ist dadurch gekennzeichnet, daß der Aktivkohleträger vor dem Aufbringen der katalytisch aktiven Komponenten, Promotoren und Modifikatoren einer oxidativen Vorbehandlung mit einer wäßrigen Lösung eines Oxidationsmittels unterzogen wird.

Als katalytisch aktive Komponenten können Edelmetalle aus der Platingruppe oder Kombinationen davon eingesetzt werden.

Als Oxidationsmittel eignen sich Wasserstoffperoxid oder Natriumhypochlorit, bevorzugt wird Wasserstoffperoxid eingesetzt.

Es wurde überraschend gefunden, daß durch eine solche oxidative Vorbehandlung der schon mit Salpetersäure gewaschenen Aktivkohle die Leistungsschwankungen der Katalysatoren vermindert werden können. Darüber hinaus erhöht sich die Aktivität gegenüber herkömmlich präparierten Katalysatoren.

Die oxidative Vorbehandlung der Aktivkohle wird bevorzugt in Form einer Wäsche mit einer 0,1 bis 30 Gewichtsprozent Wasserstoffperoxid bzw. Natriumhypochlorit enthaltenden wäßrigen Lösung bei Raumtemperaturen für die Dauer von 10 bis 300 Minuten durchgeführt.

Die Erfindung wird im folgenden an Hand einiger Beispiele näher erläutert. Für die Beispiele wurden zwei unterschiedliche Produktionslots (Lot 1 und Lot 2) einer kommerziell erhältlichen mit Salpetersäure gewaschenen Aktivkohle aus Buchenholz mit einer BET-Oberfläche von ca. 900 - 1000 m²/g, einem Gesamtporenvolumen von 0,9 - 1,0 ml/g und einer mittleren Korngröße von 23 µm eingesetzt. Der Aschegehalt der Aktivkohle betrug weniger als 2 %.

### Vergleichsbeispiel 1

99,8 g der Aktivkohle (Trockengewicht von Lot 1) wurden mit einer 20 Gew.-%igen wäßrigen Tetrachloropalladiumsäurelösung imprägniert und auf 80° C erwärmt. Dann wurde durch Fällung mit einer 10 Gew.-%igen wäßrigen NaOH-Lösung und Reduktion mit Formaldehyd bei 80° C die gewünschte metallische Phase eingestellt. Anschließend wurde der Katalysator abfiltriert und mit Wasser gewaschen. Der fertige Katalysator enthielt 5 Gew.-% Palladium auf Aktivkohle.

### Vergleichsbeispiel 2

Es wurde ein zweiter Katalysator völlig analog zu Vergleichsbeispiel 1 unter Verwendung von Aktivkohle aus Lot 2 präpariert.

### Beispiele 1 und 2

130 g der in den Vergleichsbeispielen 1 und 2 angegebenen Aktivkohle (Trockengewicht) (Lot 1 für Beispiel 1 und Lot 2 für Beispiel 2) wurden in 800 ml H₂O vorgelegt. Zu dieser Suspension wurden 50 ml einer 30 %igen H₂O₂-Lösung gegeben und anschließend wurde vier Stunden lang gerührt. Nach Abfiltrieren wurde die Aktivkohle mit Wasser gewaschen.

Die Herstellung der Katalysatoren erfolgte dann wie in den Vergleichsbeispielen.

### Katalysatortestung

Zur Messung der Katalysatorleistung wurde die Katalysatoraktivität in der Hydrierreaktion von Zimtsäure zu Dihydrozimtsäure untersucht.

Die Katalysatoraktivität wurde dabei in Milliliter verbrauchtem Wasserstoff pro Gramm Katalysator und Minute (mlH₂/g Katalysator · min) gemessen. Als Reaktionszeitraum für die Berechnung dieser Katalysatoraktivität diente die Reaktionszeit zwischen der dritten und achten Minute nach Beginn der Wasserstoffzufuhr.

In einem 250 ml Rührreaktor mit Begasungsrührer, Thermometer und Wasserstoffzufuhr wurde eine Reaktionslösung aus 10 g Zimtsäure und 200 mg Katalysator in einer Mischung aus 80 ml Wasser und 40 ml Ethanol vorgelegt. Der für die Hydrierung notwendige Wasserstoff wurde bei einer Reaktionstemperatur von 25° C, einem Wasserstoff-Überdruck von 10 mbar über normal und einer Rührgeschwindigkeit des Begasungsrührers von 2.000 min⁻¹ in der Reaktionslösung verteilt.

In Tabelle 1 sind die gemessenen Aktivitätswerte der gemäß den Beispielen und Vergleichsbeispielen präparierten Katalysatoren aufgelistet.

**Tabelle 1**

| Katalysatoraktivitäten | | | |
|---|---|---|---|
| Katalysator | Aktivkohle | H₂O₂-Waschung | Aktivität [ml/g·min] |
| Vergl.Bsp. 1 | Lot 1 | nein | 35 |
| Beispiel 1 | Lot 1 | ja | 62 |
| Vergl.Bsp. 2 | Lot 2 | nein | 82 |
| Beispiel 2 | Lot 2 | ja | 108 |

Durch die oxidative Vorbehandlung erhöht sich die Katalysatoraktivität im Falle von Aktivkohle Lot 1 von 35 auf 62 mlH₂/g min und im Falle von Aktivkohle Lot 2 von 82 auf 108 mlH₂/g min.

Zur Aufklärung dieser unerwarteten Aktivitätsstetgerungen wurden Oberflächenanalysen der Katalysatoren mit XPS (X-Ray Photoelectron Spectrometry vorgenommen. Es wurde gefunden, daß die mit Salpetersäure gewaschenen Aktivkohlen neben den gewünschten aktiven C/O-funktionellen Gruppen infolge der Salpetersäurewäsche Amin-, Ammonium- und Nitrit/Nitrat-Gruppen aufweisen.

Durch die oxidative Vorbehandlung mit Wasserstoffperoxid wird die Oberflächenkonzentration dieser Stickstoffhaltigen funktionellen Gruppen unter die Nachweisgrenze der XPS-Messung von 0,1 Flächenprozenten vermindert. Wie die Beispiele zeigen, wird dieser Effekt schon mit einer Behandlungsdauer von vier Stunden erreicht.

Tabelle 2 zeigt die Ergebnisse von XPS-Messungen an den Aktivkohleträgern vor und nach der Wasserstoffperoxid-Waschung sowie nach der Palladium-Imprägnierung. Die Oberflächenkonzentrationen des Stickstoffs wurden durch Integration der N1s-Signale gewonnen.

Bei den mit Wasserstoffperoxid gewaschenen Trägern bzw. Katalysatoren liegt die Stickstoff-Konzentration an der Oberfläche jeweils unter der Nachweisgrenze von 0,1 Flächenprozenten. Die gefundenen Aktivitätsstetgerungen korrelieren gut mit den verminderten Stickstoff-Oberflächenkonzentrationen.

Ein weiterer Vorteil der Vorbehandlung der mit Salpetersäure gewaschenen Aktivkohle mit Wasserstoffperoxid ist eine zusätzliche Verminderung der Oberflächenverunreinigung mit Spuren anorganischer Verbindungen.

Bei fehlender Vorbehandlung der Aktivkohleträger mit Wasserstoffperoxid führt die Imprägnierung mit Palladium zu einer Erhöhung der Oberflächenkonzentration des Stickstoffs durch Ausschwemmen aus den Poren des Trägers. Auch die Oberflächenanteile vorher durch HNO₃-Wäsche partiell entfernter Spurenverunreinigungen wie z. B. Al, Si, Mg, Fe u. a., die von Bedeutung für die Säure/Base-Eigenschaften der Katalysatoroberfläche sein können, nehmen im Falle nicht H₂O₂-vorbehandelter Träger bei der Pd-Imprägnierung wieder zu.

Im Falle der Vorbehandlung der mit Salpetersäure gewaschenen Träger mit Wasserstoffperoxid bleibt diese Erhöhung aus. Es wurde somit ein Reinigungseffekt durch H₂O₂ sowohl bei der Entfernung stickstoff-funktioneller Oberflächengruppen als auch anderer Oberflächenkontaminationen festgestellt.

Auf fertigen Pd/C-Katalysatoren auf Basis von HNO₃/H₂O₂-konditionierter Trägerkohlen wurden in allen Fällen sehr ähnliche XPS-Peakintensitäten gemessen, d. h. es wurde eine bessere Konstanz der Oberflächenkonzentrationen bzw. Dispersion des Edelmetalls erzielt. Dieses war bei Pd/C-Katalysatoren, die unter Verwendung der nur HNO₃-gewaschenen Trägerkohlen hergestellt wurden, nicht der Fall. Hier waren größere Los zu Los Variationen der Pd-Intensitäten festzustellen.

Diese Untersuchungen zeigen, daß die XPS-Messungen ein geeignetes Mittel sind, um den Erfolg der oxidativen Vorbehandlung mit Wasserstoffperoxid zu kontrollieren.

**Tabelle 2**

| Oberflächenkonzentration der stickstoffhaltigen funktionellen Gruppen | | | |
|---|---|---|---|
| Katalysator | Aktivkohle | H₂O₂-Waschung | Oberflächenkonzentration an Stickstoff (Atomprozent) |
| | Lot 1 | nein | 0,18 |
| | Lot 1 | ja | -^{*)} |
| | Lot 2 | nein | 0,89 |
| | Lot 2 | ja | -*⁾ |
| Vergl.Bsp. 1 | Lot 1 | nein | 0,73 |
| Beispiel 1 | Lot 1 | ja | -*⁾ |
| Vergl.Bsp. 2 | Lot 2 | nein | 1,53 |
| Beispiel 2 | Lot 2 | ja | -*⁾ |

| | | | |
|---|---|---|---|
| *⁾ unterhalb der Nachweisgrenze | | | |

## Patentansprüche

1. Verfahren zur Herstellung edelmetallhaltiger Hydrierkatalysatoren enthaltend wenigstens eine katalytisch aktive Komponente, sowie gegebenenfalls Promotoren und/oder Modifikatoren, auf einem Träger aus mit Salpetersäure gewaschener Aktivkohle,
**dadurch gekennzeichnet,**
daß der Aktivkohleträger vor dem Aufbringen der katalytisch aktiven Komponenten, Promotoren und Modifikatoren einer oxidativen Vorbehandlung mit einer wäßrigen Lösung eines Oxidationsmittels unterzogen wird.

2. Verfahren nach Anspruch 1,
**dadurch gekennzeichnet,**
daß als katalytisch aktive Komponenten Edelmetalle aus der Platingruppe oder Kombinationen davon eingesetzt werden.

3. Verfahren nach Anspruch 1,
**dadurch gekennzeichnet,**
daß das Oxidationsmittel Wasserstoffperoxid ist.

## Claims

1. A process for preparing hydrogenation catalysts which contain noble metals containing at least one catalytically active component, and optionally promoters and/or modifiers, on a support of activated carbon washed with nitric acid, characterised in that the activated carbon support is subjected to an oxidative pretreatment with an aqueous solution of an oxidising agent before application of the catalytically active components, promoters and modifiers.

2. A process according to Claim 1, characterised in that noble metals from the platinum group or combinations thereof are used as catalytically active components.

3. A process according to Claim 1, characterised in that the oxidising agent is hydrogen peroxide.

## Revendications

1. Procédé de préparation de catalyseurs d'hydrogénation à base de métaux précieux, contenant au moins un composant catalytiquement actif, ainsi qu'éventuellement des promoteurs et/ou des modificateurs, sur un support de charbon actif lavé à l'acide nitrique, caractérisé en ce que le support de charbon actif, avant l'introduction des composants catalytiquement actifs, promoteurs et modificateurs, est soumis à un prétraitement oxydant avec une solution aqueuse d'un agent d'oxydation.

2. Procédé selon la revendication 1, caractérisé en ce que, comme composants catalytiquement actifs, on fait appel à des métaux précieux du groupe du platine ou à des combinaisons de ceux-ci.

3. Procédé selon la revendication 1, caractérisé en ce que l'agent d'oxydation est du peroxyde d'hydrogène.
